# EUROPEAN PATENT APPLICATION

(11) **EP 4 545 648 A1**
(43) Date of publication of application: **30.04.2025**
(21) Application number: 23831818.2
(22) Date of filing: 23.06.2023
(51) Int. Cl.: C12Q 1/6848

(54) **METHOD FOR AMPLIFYING TARGET NUCLEIC ACID USING BETA-HYDROXY ACID**

(30) Priority: 27.06.2022 KR 20220078181
(71) Applicant: Seegene, Inc., Seoul 05548 (KR)
(72) Inventor: AHN, Byung Jun, Seoul 05548 (KR); KIM, Myung Il, Seoul 05548 (KR); LEE, Jae Min, Seoul 05548 (KR); LEE, Sang Hyuk, Seoul 05548 (KR)
(74) Representative: Dehns
(86) International application number: PCT/KR2023/008737
(87) International publication number: WO 2024/005458

(57) **Abstract**

The present disclosure relates to a method, particularly a loop-mediated isothermal amplification (LAMP) method for amplifying a target nucleic acid using beta-hydroxy acid. The beta-hydroxy acid used in the method of the present disclosure can improve the amplification rate of a target nucleic acid to reduce the time required for a LAMP reaction as well as inhibit non-specific amplification to reduce the possibility of false positives.

## Description

### [Technical Field]

### Cross-Reference To Related Applications

This application claims priority from Korean Patent Application No. 10-2022-0078181, filed on June 27, 2022, in the Korean Intellectual Property Office, the disclosure of which is incorporated herein by reference in its entirety.

### Technical Field

The present disclosure relates to a method, particularly a loop-mediated isothermal amplification (LAMP) method for amplifying a target nucleic acid using beta-hydroxy acid.

### [Background Art]

Loop mediated isothermal amplification (LAMP) is a method developed by Eiken Chemical Co., Ltd., which is characterized by using four or six oligonucleotide primers to enhance the rate (see US 6410278B1). The LAMP method employs an enzyme having high strand displacement activity and, unlike PCR, is performed at isothermal conditions (typically about 60-65°C). LAMP has a fast reaction rate and high specificity and does not require expensive equipment compared to PCR, due to performance at an isothermal temperature. However, the LAMP method requires complex primer design and a large amount of primers compared to PCR, which may cause false positives due to non-specific amplification in negative samples that do not contain target nucleic acids. The cause of this problem may include carry-over contamination due to high amplification efficiency of LAMP in addition to the unique characteristics of the primers used in the LAMP reaction, but the exact cause has not yet been identified.

Meanwhile, LAMP requires an enzyme having strand displacement activity to form loop structures and to amplify along continuously repeating loops. To date, *Bst* DNA polymerase is known as the most suitable enzyme. This enzyme was first purified in 1972 from *Geobacillus stearothermophilus,* which survives at 60-70°C (see Setnesh and Roe Biochim. Biophys. Acta 1972, 272(2), 156-166), and belongs to the Family A DNA polymerase. It shares about 45% homology to *Taq* DNA polymerase generally used in PCR. Later, it was found that a large fragment produced by digestion of the enzyme with subtilisin lacks 5' to 3' exonuclease activity and has high strand displacement activity (see J. M. Aliotta et al. Genet Anal. 1996, 12(5-6), 185-195). While the *Bst* DNA polymerase has 876 amino acids in length, the large fragment as described above has 587 amino acids in length. Thereafter, the large fragment of *Bst* DNA polymerase (*Bst* DNA polymerase, large fragment) was utilized as a main enzyme in the LAMP method based on strand displacement by Eiken Chemical Co., Ltd.

This enzyme is suitable for application to LAMP due to high strand displacement and lack of 5' to 3' exonuclease activity, but its low stability against salt and dUTP makes it unfavorable for use in target genes with various GC contents or for application to UDG system, and its reaction rate is also rather slow. In addition to the large fragment of *Bst* DNA polymerase, enzymes capable of performing LAMP reaction have been reported, including *Bsm* (*Bacillus smithii*) DNA polymerase large fragment (Thermo Scientific), *Bsu* (*Bacillus subtilis*) DNA polymerase large fragment (NEB), *Sau* (*Staphylococcus aureus*) DNA polymerase large fragment (Intact Genomics), and engineered versions of *Taq* polymerase (SD polymerase - BIORON GmbH, D732N mutated *Taq* - DNA Polymerase Technology Inc.), but these enzymes are not widely used compared to the *Bst* DNA polymerase because they require a change in reaction conditions or have low efficiency.

The LAMP method as described above has recently emerged as one of the methods for detecting SARS-CoV-2 due to its speed, simplicity, and no need for complex equipment compared to conventional PCR. However, LAMP has non-specific amplification due to the use of complex primers and limited enzymatic capacity of *Bst* DNA polymerase. Therefore, in order to appropriately utilize LAMP for molecular diagnostic purposes, it is required to develop a technology that overcomes this limitation.

Until now, in order to improve non-specific amplification, *i.e.*, in order to reduce a non-specific amplification signal, a method of identifying the occurrence of primer dimers and changing the sequences of primers involved in the occurrence of the primer dimers has been used, but it requires a lot of time and effort. In addition, in order to improve the limitation of the capacity of *Bst* DNA polymerase, attempts have been made to engineer this enzyme to improve the polymerization rate, increase its tolerance to salt or heat, or increase its tolerance to dUTP (K. Hsieh et al. Chem. Commun., 2014, 50, 3747-3749.), but these require excessive time and effort for research and development, and above all, redesign of the product formulation and conditions to newly set up buffers and reaction conditions suitable for the engineered enzyme.

Therefore, there remains a need in the art to develop a method capable of improving the amplification rate and non-specific amplification of LAMP without changing the sequence of primers or enzymatic engineering.

Throughout this specification, a number of patents and documents are referenced and their references are indicated in parentheses. In order to more clearly describe the present disclosure and the level of the technical field to which the present disclosure pertains, the disclosures of these patents and documents are incorporated herein by reference in their entirety.

### [Disclosure]

### [Technical Problem]

The present inventors endeavored to develop a method capable of improving the reaction rate and reducing non-specific amplification signals in a LAMP reaction. As a result, the present inventors have found that the addition of beta-hydroxy acid to a LAMP reagent can improve the reaction rate of LAMP and address the false positive problem caused by non-specific amplification.

Accordingly, it is an object of the present disclosure to provide a method for amplifying a target nucleic acid using a LAMP reagent comprising beta-hydroxy acid.

It is another object of the present disclosure to provide a LAMP reagent comprising beta-hydroxy acid.

It is still another object of the present disclosure to provide a method for reducing a non-specific amplification signal in amplification of a target nucleic acid.

### [Technical Solution]

According to an aspect of the present disclosure, there is provided a method for amplifying a target nucleic acid, the method comprising: (a) providing a loop-mediated isothermal amplification (LAMP) reagent, the reagent comprising: (i) a set of primers comprising an F3 primer, a B3 primer, a forward inner primer (FIP), and a backward inner primer (BIP); (ii) a nucleic acid polymerase; (iii) dNTPs; (iv) beta-hydroxy acid; and (v) a buffer; and (b) incubating the LAMP reagent with a target nucleic acid at an isothermal temperature to amplify the target nucleic acid.

In an embodiment, the beta-hydroxy acid is a bidentate chelator compound.

In an embodiment, the beta-hydroxy acid is selected from the group consisting of serine, carnitine, threonine, and combinations thereof.

In an embodiment, each of the serine, carnitine and threonine is in an L-isomeric form.

In an embodiment, the beta-hydroxy acid is L-serine, and the L-serine is comprised at a concentration of 15 to 80 mM.

In an embodiment, the beta-hydroxy acid is L-carnitine, and the L-carnitine is comprised at a concentration of 0.2 to 20 mM.

In an embodiment, the beta-hydroxy acid is L-threonine, and the L-threonine is comprised at a concentration of 15 to 80 mM.

In an embodiment, the beta-hydroxy acid is a combination of L-serine and L-carnitine.

In an embodiment, the nucleic acid polymerase has strand displacement activity and lacks 5' to 3' exonuclease activity.

In an embodiment, the nucleic acid polymerase is selected from the group consisting of a full-length or large fragment of *Bst (Bacillus stearothermophilus)* DNA polymerase, a full-length or large fragment of *Bsm (Bacillus smithii)* DNA polymerase, a full-length or large fragment of *Bsu (Bacillus subtilis)* DNA polymerase, a full-length or large fragment of *Sau* (*Staphylococcus aureus*) DNA polymerase, and a mutant thereof.

In an embodiment, the set of primers further comprises one or both of a loop forward (LF) primer and a loop backward (LB) primer.

In an embodiment, the method increases amplification efficiency of the target nucleic acid while reducing non-specific amplification as compared to using a LAMP reagent that does not include beta-hydroxy acid.

In an embodiment, the target nucleic acid is 3 to 5.

In an embodiment, the target nucleic acid is RNA.

In an embodiment, the method further comprises reverse transcribing the target RNA into target DNA.

According to another aspect, there is provided a loop mediated isothermal amplification (LAMP) reagent, the reagent comprising: (i) a set of primers comprising an F3 primer, a B3 primer, a forward inner primer (FIP), and a backward inner primer (BIP); (ii) a nucleic acid polymerase; (iii) dNTPs; (iv) beta-hydroxy acid; and (v) a buffer.

In an embodiment, the beta-hydroxy acid is a bidentate chelator compound.

In an embodiment, the beta-hydroxy acid is selected from the group consisting of serine, carnitine, threonine, and combinations thereof.

In an embodiment, the serine, carnitine and threonine are in an L-isomeric form.

In an embodiment, the beta-hydroxy acid is L-serine, and the L-serine is comprised at a concentration of 15 to 80 mM.

In an embodiment, the beta-hydroxy acid is L-carnitine, and wherein the L-carnitine is comprised at a concentration of 0.2 to 20 mM.

In an embodiment, the beta-hydroxy acid is L-threonine, and the L-threonine is comprised at a concentration of 10 to 50 mM.

In an embodiment, the beta-hydroxy acid is a combination of L-serine and L-carnitine.

In an embodiment, the nucleic acid polymerase has strand displacement activity and lacks 5' to 3' exonuclease activity.

In an embodiment, the nucleic acid polymerase is selected from the group consisting of a full-length or large fragment of *Bst* (*Bacillus stearothermophilus*) DNA polymerase, a full-length or large fragment of *Bsm* (*Bacillus smithii*) DNA polymerase, a full-length or large fragment of *Bsu* (*Bacillus subtilis*) DNA polymerase, a full-length or large fragment of *Sau* (*Staphylococcus aureus*) DNA polymerase, and a mutant thereof.

In an embodiment, the primer set further comprises one or both of a loop forward (LF) primer and a loop backward (LB) primer.

In an embodiment, the reagent increases the amplification rate of the target nucleic acid while reducing non-specific amplification as compared to a LAMP reagent not containing beta-hydroxy acid.

In an embodiment, the target nucleic acid is 3 to 5.

In an embodiment, the target nucleic acid is RNA.

According to another aspect, there is provided a method for reducing a non-specific amplification signal in amplification of a target nucleic acid, the method comprising: (a) providing a loop-mediated isothermal amplification (LAMP) reagent, the reagent comprising: (i) a set of primers comprising an F3 primer, a B3 primer, a forward inner primer (FIP), and a backward inner primer (BIP); (ii) a nucleic acid polymerase; (iii) dNTPs; (iv) beta-hydroxy acid; and (v) a buffer; and (b) incubating the LAMP reagent with a target nucleic acid at an isothermal temperature to amplify the target nucleic acid, wherein the LAMP reagent comprising beta-hydroxy acid reduces non-specific amplification as compared to a LAMP reagent not containing beta-hydroxy acid.

### [Advantageous Effects]

The beta-hydroxy acid used in the method of the present disclosure can improve the amplification rate of a target nucleic acid to reduce the time required for a LAMP reaction as well as inhibit non-specific amplification to reduce the possibility of false positives.

### [Description of Drawings]

FIG. 1 shows amplification curves, which were each obtained by subjecting a LAMP reagent not containing beta-hydroxy acid and an additive (Ex. 1-1), or a LAMP reagent comprising either citrate (Ex. 1-2), L-serine (Ex. 1-3), L-carnitine (Ex. 1-4), betaine (Ex. 1-5), DMSO (Ex. 1-6), or a combination of FA and DMF (Ex. 1-7) to a reaction with a target nucleic acid.
FIG. 2 shows Ct values (Time to Positive) calculated from amplification curves for LAMP reagents of FIG. 1.
FIG. 3 shows Ct values (Time to Positive) calculated from amplification curves, which were each obtained by subjecting a LAMP reagent not containing beta-hydroxy acid and citrate (Ex. 2-1), or a LAMP reagent comprising either 0.2 M citrate (Ex. 2-2), 0.02 M citrate (Ex. 2-3), 0.2 M L-serine (Ex. 2-4), 0.02 M L-serine (Ex. 2-5), 0.2 M L-carnitine (Ex. 2-6), 0.02 M L-carnitine (Ex. 2-7), or 0.002 M L-serine (Ex. 2-8) to a reaction with no template control (NTC).
FIG. 4 shows Ct values of target nucleic acids (left bar at each concentration) or NTCs (right bar at each concentration) using LAMP reagents each containing 0, 10, 20, 50, 100, or 200 mM L-serine.
FIG. 5 shows difference (fold increase relative to 0 mM) between Ct value of target nucleic acids and Ct value of NTCs using LAMP reagents each containing 0, 10, 20, 50, 100, or 200 mM L-serine.
FIG. 6 shows Ct values of target nucleic acids (left bar at each concentration) or NTCs (right bar at each concentration) using LAMP reagents each containing 0, 0.5, 1, 10, or 20 mM L-carnitine.
FIG. 7 shows difference (fold increase relative to 0 mM) between Ct value of target nucleic acids and Ct value of NTCs using LAMP reagents each containing 0, 0.5, 1, 10, or 20 mM L-carnitine.

### [Best Mode]

### I. Method for amplifying target nucleic acid

According to an aspect of the present disclosure, there is provided a method for amplifying a target nucleic acid, the method comprising:
(a) providing a loop-mediated isothermal amplification (LAMP) reagent, the reagent comprising:
   (i) a set of primers comprising an F3 primer, a B3 primer, a forward inner primer (FIP), and a backward inner primer (BIP);
   (ii) a nucleic acid polymerase;
   (iii) dNTPs;
   (iv) beta-hydroxy acid; and
   (v) a buffer; and
(b) incubating the LAMP reagent with a target nucleic acid at an isothermal temperature to amplify the target nucleic acid.

Hereinafter, the present invention will be described in more detail.

According to the present disclosure, the method is used to amplify a target nucleic acid.

As used herein, the term "nucleic acid," "nucleic acid sequence," or "nucleic acid molecule" refers to a deoxyribonucleotide or ribonucleotide polymer in single-stranded form or double-stranded form, and the nucleotide may include derivatives, non-natural nucleotides, or modified nucleotides of natural nucleotides that can function in the same manner as natural nucleotides.

As used herein, the term "target nucleic acid," "target nucleic acid sequence," or "target sequence" refers to a nucleic acid sequence to be detected. The target nucleic acid may be hybridized with some primers or probes included in the LAMP reagent used in the method of the present disclosure.

According to an embodiment, the target nucleic acid may be a nucleic acid from a human, an animal, a plant, or a microorganism. The microorganism may be fungi, protozoa, bacteria, viruses, or algae.

According to an embodiment, the target nucleic acid may be a viral nucleic acid, and specifically, the target nucleic acid may be an RNA virus nucleic acid.

According to an embodiment, the target nucleic acid may be a respiratory virus nucleic acid. For example, the respiratory virus nucleic acid includes, but is not limited to, influenza virus nucleic acid, respiratory syncytial virus (RSV) nucleic acid, adenovirus nucleic acid, enterovirus nucleic acid, parainfluenza virus nucleic acid, metapneumovirus (MPV) nucleic acid, bocavirus nucleic acid, rhinovirus nucleic acid, and/or coronavirus nucleic acid.

According to an embodiment, the target nucleic acid may be a Severe acute respiratory syndrome coronavirus 2 (SARS-CoV-2) virus nucleic acid.

According to an embodiment, the SARS-CoV-2 virus nucleic acid may be selected from the group consisting of an E gene, an N gene, an RdRP gene, an S gene, and combinations thereof.

In an embodiment, the target nucleic acid is 3 to 5. Three to five target nucleic acids may be simultaneously amplified and detected by the LAMP reagent according to the present disclosure in one reaction vessel.

The target nucleic acid as described above may be present in a sample from a subject.

As used herein, the term "subject" refers to an individual suspected of containing a target nucleic acid *(e.g.,* a nucleic acid of a particular pathogen) to be detected using the methods of the present disclosure. Examples of the subject include, but are not limited to, mammals such as dogs, cats, rodents, primates, humans, and the like, and in particular, humans.

As used herein, the term "sample" refers to any analyte containing or suspected of containing a nucleic acid to be detected. For example, the sample includes a biological sample *(e.g.,* cells, tissues, and body fluids) and an non-biological sample *(e.g.,* food, water, and soil), and the biological sample may be, for example, viruses, bacteria, tissues, cells, blood (including whole blood, plasma, and serum), lymph, bone marrow fluid, saliva, sputum, swabs, aspiration fluid, milk, urine, feces, ocular fluid, semen, brain extracts, spinal fluid, joint fluid, thymus fluid, bronchial lavage fluid, ascites, or amniotic fluid, but is not limited thereto.

The sample may be used interchangeably with "specimen" herein.

According to an embodiment, the sample may be obtained from a subject, particularly a mammal, more particularly a human, and may be, for example, a swab, saliva, sputum, aspiration, bronchoalveolar lavage (BAL), gargle, or blood, but is not limited thereto.

In certain embodiments, the sample derived from the subject is a swab, saliva, or a mixture thereof.

In an embodiment, the swab is nasopharyngeal swab, nostril swab, oropharyngeal swab, oral swab, saliva swab, genital swab, rectal swab, or a combined swab of two or more thereof.

### Step (a): Provision of a LAMP reagent

According to step (a) of the present disclosure, a loop-mediated isothermal amplification (LAMP) reagent is provided.

The LAMP reagent is used for amplifying a target nucleic acid by the LAMP method and, optionally, generating a signal indicating the presence of the target nucleic acid from the amplification product of the target nucleic acid.

As used herein, the term "loop-mediated isothermal amplification (LAMP)" refers to one of the methods of amplifying a target nucleic acid under isothermal conditions, unlike conventional polymerase chain reaction (PCR) (Notomi, T. et al. 2000. Loop-Mediated Isothermal Amplification of DNA. Nucleic Acids Res 28, E63).

The LAMP reagent used in the method according to the present disclosure refers to all components included in the LAMP reaction, except for components derived from a sample, such as a target nucleic acid.

The LAMP reagent according to the present disclosure comprises: (i) a set of primers comprising an F3 primer, a B3 primer, a forward inner primer (FIP), and a backward inner primer (BIP); (ii) nucleic acid polymerase; (iii) dNTPs; (iv) beta-hydroxy acid; and (v) a buffer.

### (i) Set of primers

The LAMP reagent comprises a set of primers set consisting of a plurality of primers.

As used herein, the term "primer" refers to a single-stranded oligonucleotide used in an amplification reaction, which is a nucleic acid molecule that is extended by addition of nucleotides to its 3' end by a covalent bond in a nucleic acid amplification or synthesis reaction using a polymerase.

The set of primers in the LAMP reagent according to the present method comprises four types of primers (F3, B3, FIP, and BIP) known in the art. The four primers are composed of two outer primers and two inner primers. The outer primers are composed of two forward outer (F3) and two backward outer (B3) primers and serves to unwind double stranded DNA during a non-cyclic step of the reaction. The internal primers are composed of two types, *i.e.*, a forward inner primer (FIP) and a backward inner primer (BIP), which are composed of nucleotides corresponding to forward and reverse nucleotide sequences so as to form a loop essential for the LAMP reaction.

In an embodiment, the set of primers further comprises either or both of a loop forward (LF) primer and a loop backward (LB) primer. The LF primer and the LB primer are attached to a nucleotide sequence to which the inner primer does not bind, thereby accelerating the LAMP reaction.

The set of primers as described above is designed for six distinct regions (F3, F2, F1, B1c, B2c, and B3) on the target nucleic acid, in which as two outer primers, F3 and B3 determine the size of the product to be amplified and as two inner primers, FIP consists of F1c and F2 sequences, and BIP consists of B1c and B2 sequences.

The LAMP reaction largely comprises a starting structure producing step and a cycling amplification step, and does not include a step of denaturation into a single strand, unlike PCR. In the starting structure producing step, F2 of FIP binds to the F2c region to initiate elongation. The BIP also proceeds in the same manner. The F3 primer binds to the F3c region and initiates DNA synthesis in a strand displacement manner to replace and release the DNA strand elongated in the FIP. The released single strand forms a loop at its 5' end through F1/F1c bond, and the BIP and B3 primers also undergo a synthesis reaction in the same manner, thereby forming a single strand nucleic acid molecule having loops of the dumbbell shape at both ends. Subsequently, DNA synthesis starts from the 3' end of the F1 region, and the cycling step proceeds.

LB or LF primers, which are acceleration primers, bind to a single-stranded region of the dumbbell structure described above and bind to a loop between B1 and B2 or between F1 and F2. The use of LB or LF primers increase the origin at which DNA synthesis begins, resulting in a faster reaction rate. The LAMP reaction usually forms six loops in its process, and only two loops are used as a synthesis origin, but in the case of using LB and LF primers, all six loops are used as a synthesis origin, so that the amount of DNA synthesized for a predetermined time increases.

A detailed description of the features and functions of each of the F3 primer, the B3 primer, the FIP primer, the BIP primer, the Loop F (LF) primer, and the Loop B (LB) primer can be found in T. Notomi, H. Okayama, H. Masubuchi, T. Yonekawa, K. Watanabe, N. Amino and T. Hase, Nucleic Acids Res., 2000, 28, E63 and K. Nagamine, T. Hase and T. Notomi, Mol. Cell. Probes, 2002, 16, 223-229.

According to an embodiment of the present disclosure, a suitable length of the primer may be determined in consideration of a plurality of factors, for example, temperature, application, a source of the primer, a length of a final amplification product, and the like. For example, the length of the primer may be at least 10 nucleotides, and F3 and B3 may be at least 15 nucleotides in length, FIP and BIP may be at least 30 nucleotides in length, and LF and LB may be at least 15 nucleotides in length, but is not limited thereto.

According to an embodiment of the present disclosure, the set of primers may be designed using various commercially available primer design software, such as Primer Explorer V5 (Fujitsu, Japan), LAVA (LAMP Assay Versatile Analysis), and LAMP Designer (PRIMER Biosoft), in consideration of the characteristics of the LAMP reaction and the primers.

For instance, six primers having nucleotide sequences of SEQ ID NOs: 1 to 6 were used to M13mp18 single stranded circular DNA in Example of the present disclosure (see Schneider et al. Electrophoresis 2019, 40, 2706-2717).

### (ii) Nucleic acid polymerase

The LAMP reagent according to the methods of the present disclosure comprises a nucleic acid polymerase, particularly a DNA polymerase.

According to an embodiment of the present disclosure, the nucleic acid polymerase may be one that (i) has strand displacement activity and (ii) causes a polymerization reaction at a temperature (e.g., between 50°C and 75°C) at which its thermodynamic activity is maintained so that a polymer which is a reaction product can form a loop structure. Examples thereof include *Thermus thermophilus* (*Tth*) DNA polymerase, *Thermus aquaticus* (*Taq*) DNA polymerase, *Thermococcus litoralis* DNA polymerase; *Pyrococcus furiosus* (*Pfu*) DNA polymerase, or *Bacillus caldotenax* DNA polymerase.

In one embodiment, the nucleic acid polymerase comprises one which has strand displacement activity and lacks 5' to 3' exonuclease activity. Examples thereof may include a full-length or large fragment of *Bst* (*Bacillus stearothermophilus*) DNA polymerase, a full-length or large fragment of *Bsm* (*Bacillus smithii*) DNA polymerase, a full-length or large fragment of *Bsu* (*Bacillus subtilis*) DNA polymerase, a full-length or large fragment of *Sau* (*Staphylococcus aureus*) DNA polymerase, and a mutant thereof. The mutant may include an insertion or deletion in the amino acid sequence of the DNA polymerase described above.

In certain embodiments, *Bst* DNA polymerase, large fragment of SEQ ID NO: 14 as disclosed in U.S. Pat. No. 5814506A, *Bst* 2.0 DNA polymerase (NEB) having improved reaction rate or the like, or a product in the form of LAMP Mastermix having improved convenience may be used as the nucleic acid polymerase.

In addition, for the RT-LAMP reaction, GspSSD DNA polymerase having a faster amplification capability and a stronger reverse transcriptase activity than *Bst* DNA polymerase mainly used in the conventional LAMP reaction may be used, but is not limited thereto.

### (iii) dNTPs

The LAMP reagent according to the methods of the present disclosure comprises dNTPs. dNTP refers to a nucleotide that serves as a substrate upon synthesis of DNA by polymerase. The dNTPs may include dATP, dCTP, dGTP, and dTTP, and may be commercially available dNTP Mix or may be a mixture of commercially available individual dATP, dCTP, dGTP, and dTTP.

According to an embodiment of the present disclosure, the LAMP reagent may further comprise a nucleotide analog instead of dNTPs. A nucleotide analog is one that is modified or not found in nature and can be polymerized alone or in combination with naturally occurring nucleotides in a template-directed DNA synthesis process.

### (iv) Beta-hydroxy acid

The LAMP reagent according to the methods of the present disclosure comprises beta-hydroxy acid.

As used herein, the term "beta-hydroxy acid" collectively refers to an organic compound containing a carboxyl group and a hydroxyl group separated by two carbon atoms. The beta-hydroxy acid was named based on the attachment of the hydroxyl group to the beta-position of the carboxyl group. Examples of the beta-hydroxy acid include, without limitation, salicylic acid, beta hydroxybutanoic acid, tropic acid, tratocanoic acid, serine, carnitine, threonine, and the like.

The beta-hydroxy acid having the hydroxyl group at the beta-position of the carboxyl group is distinct from the alpha-hydroxy acid having the hydroxyl group at the alpha-position of the carboxyl group.

According to the present disclosure, the beta-hydroxy acid has the effect of improving the amplification rate of the target nucleic acid and suppressing non-specific amplification in the LAMP reaction.

Conventionally, in order to improve the LAMP method, compounds such as betaine or DMSO, or enzymes that destabilize the double-stranded structure of the nucleic acids to prevent amplification of non-specific nucleic acids have been mainly used. Such compounds or enzymes effectively inhibit non-specific amplification, but also inhibit formation of a double strand by hybridization between a primer and a target nucleic acid, resulting in reduced amplification of target nucleic acids, *i.e.,* reduced reaction rate of target nucleic acids.

On the other hand, the beta-hydroxy acid used in the present disclosure does not inhibit the amplification of the target nucleic acid while inhibiting non-specific amplification.

Without wishing to be bound by any particular theory, it is thought that such effect of the beta-hydroxy acid arises from its action as a weak chelator for Mg²⁺, which is required for nucleic acid amplification.

It is known that Mg²⁺ enhances the activity of a polymerase, *i.e.,* increases the rate of amplification of target nucleic acids and increases the Tm value of the double-stranded DNA. Further, it is known that low concentrations of Mg²⁺ interfere with amplification of the targets, whereas high concentrations of Mg²⁺ increase non-specific amplification and background.

In this regard, it is known that a strong chelator such as ethylene-diamine-tetraacetic acid (EDTA) binds to Mg²⁺ to reduce free Mg²⁺, thereby suppressing amplification of target nucleic acids in a LAMP reaction.

Based on this fact, the present inventors have focused on beta-hydroxy acid as a weak chelator for Mg²⁺.

Beta-hydroxy acid is expected to exhibit weaker binding affinity for Mg²⁺ than EDTA, nitrilotriacetic acid (NTA), and citric acid known in the art. This is because EDTA is a hexadentate ligand having a denticity (the number of functional groups in the molecule that can bind to one ligand in coordination chemistry) of 6, NTA is a tetradentate ligand having a denticity of 4, and citric acid is a tridentate ligand having a denticity of 3, whereas beta-hydroxy acid is a bidentate ligand having a denticity of 2.

Beta-hydroxy acid, which is a weak chelator, is expected to weakly bind to Mg²⁺ at the beginning of the reaction to interfere with non-specific amplification and then release free Mg²⁺ in the middle of the reaction to improve amplification of the target.

In an embodiment, the beta-hydroxy acid is a bidentate chelator compound.

In one embodiment, the beta-hydroxy acid does not comprise a compound that is also classified as alpha-hydroxy acid. For example, citric acid has a hydroxyl group at the alpha-position of one carboxyl group and a hydroxyl group at the beta-position of another carboxyl group, so it can be classified as either alpha-hydroxy acid or beta-hydroxy acid. The beta-hydroxy acid of the present disclosure does not comprise compounds such as citric acid.

In one embodiment, the beta-hydroxy acid according to the present disclosure is selected from the group consisting of serine, carnitine, threonine, and combinations thereof.

In certain embodiments, the beta-hydroxy acid according to the present disclosure is serine or carnitine.

It is not known in the art that the beta-hydroxy acid such as serine, carnitine, threonine, *etc.* can improve amplification of target nucleic acids and inhibit non-specific amplification in a LAMP reaction.

In an embodiment, each of the serine, carnitine, and threonine is in an L-isomeric form. That is, the beta-hydroxy acid according to the present disclosure may be selected from the group consisting of L-serine, L-carnitine, L-threonine, and combinations thereof. According to additional studies by the present inventors, L-serine, L-carnitine, or L-threonine was superior to D-serine, D-carnitine, or D-threonine, respectively, in terms of improving target amplification and inhibiting non-specific amplification (data not shown).

According to the present disclosure, the beta-hydroxy acid as described above should be included in the LAMP reagent at an appropriate concentration to achieve the maximum effect.

In an embodiment, when the beta-hydroxy acid is L-serine, the L-serine may be comprised in the LAMP reagent at a concentration of 15 to 80 mM. For example, the L-serine may be comprised at a concentration of 15 to 70 mM, 15 to 60 mM, 15 to 50 mM, 15 to 40 mM, 15 to 30 mM, 15 to 20 mM, 20 to 80 mM, 20 to 70 mM, 20 to 60 mM, 20 to 50 mM, 20 to 40 mM, 20 to 30 mM, 30 to 80 mM, 30 to 70 mM, 30 to 60 mM, 30 to 50 mM, 30 to 40 mM, 40 to 80 mM, 40 to 70 mM, 40 to 60 mM, 40 to 50 mM, 50 to 80 mM, 50 to 70 mM, 50 to 60 mM, 60 to 80 mM, 60 to 70 mM, or 70 to 80 mM. In certain embodiments, the optimal concentration of L-serine may be a concentration of 20 to 50 mM. Addition of L-serine at a concentration of less than 15 mM does not show significant difference compared to no addition of L-serine, and addition of L-serine at a concentration of more than 80 mM results in inhibited amplification of target nucleic acids and increased non-specific amplification, compared to no addition of L-serine.

In an embodiment, when the beta-hydroxy acid is L-carnitine, the L-carnitine may be comprised in the LAMP reagent at a concentration of 0.2 to 20 mM. As examples, the L-carnitine may be comprised at a concentration of 0.2 to 15 mM, 0.2 to 13 mM, 0.2 to 10 mM, 0.2 to 7 mM, 0.2 to 5 mM, 0.2 to 3 mM, 0.2 to 1 mM, 0.2 to 0.5 mM, 0.5 to 20 mM, 0.5 to 15 mM, 0.5 to 13 mM, 0.5 to 10 mM, 0.5 to 7 mM, 0.5 to 5 mM, 0.5 to 3 mM, 0.5 to 1 mM, 1 to 20 mM, 1 to 15 mM, 1 to 13 mM, 1 to 10 mM, 1 to 7 mM, 1 to 5 mM, 1 to 3 mM, 3 to 20 mM, 3 to 15 mM, 3 to 13 mM, 3 to 10 mM, 3 to 7 mM, 3 to 5 mM, 5 to 20 mM, 5 to 15 mM, 5 to 13 mM, 5 to 10 mM, 5 to 7 mM, 7 to 20 mM, 7 to 15 mM, 7 to 13 mM, 7 to 10 mM, 10 to 20 mM, 10 to 15 mM, 10 to 13 mM, 13 to 20 mM, 13 to 15 mM, or 15 to 20 mM. In certain embodiments, the optimal concentration of L-carnitine may be 0.5 to 10 mM. Addition of L-carnitine at a concentration of less than 0.2 mM does not show significant difference compared to no addition of L-carnitine, and addition of L-carnitine at a concentration of more than 20 mM results in inhibited amplification of target nucleic acids and increased non-specific amplification, compared to no addition of L-carnitine.

In an embodiment, when the beta-hydroxy acid is L-threonine, the L-threonine may be comprised in the LAMP reagent at a concentration of 15 to 80 mM. As examples, the L-threonine may be comprised at a concentration of 15 to 70 mM, 15 to 60 mM, 15 to 50 mM, 15 to 40 mM, 15 to 30 mM, 15 to 20 mM, 20 to 80 mM, 20 to 70 mM, 20 to 60 mM, 20 to 50 mM, 20 to 40 mM, 20 to 30 mM, 30 to 80 mM, 30 to 70 mM, 30 to 60 mM, 30 to 50 mM, 30 to 40 mM, 40 to 80 mM, 40 to 70 mM, 40 to 60 mM, 40 to 50 mM, 50 to 80 mM, 50 to 70 mM, 50 to 60 mM, 60 to 80 mM, 60 to 70 mM, or 70 to 80 mM. In certain embodiments, the optimal concentration of L-threonine may be 20 to 50 mM. Addition of L-threonine at a concentration of less than 15 mM does not show significant difference compared to no addition of L-threonine, and addition of L-threonine at a concentration of more than 80 mM results in inhibited amplification of target nucleic acids and increased non-specific amplification, compared to no addition of L-threonine.

In an embodiment, the beta-hydroxy acid may be a combination of L-serine and L-carnitine. In an embodiment, when the beta-hydroxy acid is a combination of L-serine and L-carnitine, the L-serine may be comprised in the LAMP reagent at a concentration of 15 to 80 mM, and the L-carnitine may be comprised in the LAMP reagent at a concentration of 0.2 to 20 mM. In certain embodiments, the L-serine and L-carnitine can be at a concentration of 7.5 to 40 mM and of 0.1 to 10 mM, respectively. In certain embodiments, the L-serine and L-carnitine may be mixed at a molar ratio of 1:1 to 1:10.

### (v) Buffer

The LAMP reagent according to the present disclosure comprises a buffer.

The buffer may be any buffer known to be used in the LAMP method.

According to an embodiment of the present disclosure, example of the buffer includes, without limitation, sodium phosphate buffer, potassium phosphate buffer, Tris-HCl buffer, or Tricine buffer.

In addition to the above-described components, the LAMP reagent according to the present disclosure may further comprise other components known in the art.

In an embodiment, when the target nucleic acid is RNA, the LAMP reagent may further comprise components for a reverse transcription reaction.

A reverse transcription reaction is one of synthesizing cDNA from RNA using a reverse transcriptase. Details thereof are found in Joseph Sambrook, et al., Molecular Cloning, A Laboratory Manual, Cold Spring Harbor Laboratory Press, Cold Spring Harbor, N.Y. (2001); and Noonan, K.F. et al., Nucleic Acids Res. 16:10366 (1988).

In an embodiment, the LAMP reagent may further comprise a reverse transcriptase.

As used herein, the term "reverse transcriptase", also referred to as RNA-dependent DNA polymerase, refers to an enzyme that synthesizes complementary DNA from RNA as a template.

In the present disclosure, the reverse transcriptase may be one derived from various sources, for example, including, without limitation, Avian Myeloblastosis Virus-derived Reverse Transcriptase (AMV RTase), Murine Leukemia Virus-derived Reverse Transcriptase (MuLV RTase), and Rous-Associated Virus 2 Reverse Transcriptase (RAV-2 RTase).

Reverse transcriptase requires primers to synthesize cDNA from RNA template. There are largely three types of primers to be used in the reverse transcription reaction: (i) oligo dT primers that are annealed to poly A tail of mRNA to synthesize cDNA from the 3'-end, (ii) random primers that are made of random nucleotides having a size of 6 to 9 nt to initiate synthesis across all regions of RNA, and (iii) target specific primers that synthesize only target cDNA.

According to an embodiment of the present disclosure, the LAMP reagent may comprise a primer for reverse transcription.

According to an embodiment of the present disclosure, the primer for reverse transcription may be an oligo dT-primer, a random primer, or a target specific primer.

According to an embodiment of the present disclosure, the primer for reverse transcription may be a target specific primer.

According to an embodiment of the present disclosure, the target specific primer may be used to synthesize cDNA in a reverse transcription reaction, and then used as a pair of primers in combination of other primers to amplify the synthesized cDNA in a LAMP reaction.

The LAMP reagent according to the present disclosure may further comprise magnesium, which may be used in the form of a salt such as magnesium acetate, magnesium chloride, or magnesium sulfate.

In addition, the LAMP reagent according to the present disclosure may further comprise components for detecting an amplification product of the target nucleic acid by the LAMP method. Examples of such components include, but are not limited to, fluorescent dyes such as SYBR Green I, probe oligonucleotides labeled with fluorescent dyes (optionally quencher), and dyes that change color in response to changes in pH or magnesium ion concentration.

The LAMP reagent according to the present disclosure may be used in the form of a master mix. As used herein, the term "master mix" refers to a mixture that is designed to improve user convenience and to reduce mistakes and is particularly for use in LAMP or RT-LAMP. The master mix generally refers to a mixture of a nucleic acid polymerase, MgSO₄, dNTP, a pH buffer, and the like, except for target nucleic acids and components specific for the target nucleic acids such as a set of primers or a probe. In some instances, the set of primers may be included in the master mix, or an enzyme and a buffer may be divided in the master mix.

### Step (b): Amplification of a target nucleic acid

In step (b) of the present disclosure, the LAMP reagent is incubated with a target nucleic acid at an isothermal temperature to amplify the target nucleic acid.

The amplification of the target nucleic acid may be performed by one of various LAMP methods known in the art.

As described above, when the target nucleic acid to be detected is RNA, the method of the present disclosure may further comprise a step of reverse transcription reaction before step (b). The reverse transcription may be performed according to a method known in the art.

In an embodiment of the method of the present disclosure, the incubation for amplification of the target nucleic acid may be carried out at a temperature selected from 50 to 75°C for 10 to 30 minutes.

The temperature and time for incubation of step (b) may be readily determined by those skilled in the art in consideration of enzymes, target nucleic acids, and the like used in the reaction.

In certain embodiments, the temperature for incubation of step (b) is any temperature selected from 50°C to 70°C, any temperature selected from 50°C to 65°C, any temperature selected from 55°C to 75°C, any temperature selected from 55°C to 70°C, any temperature selected from 55°C to 65°C, any temperature selected from 60°C to 75°C, any temperature selected from 60°C to 70°C, or any temperature selected from 60°C to 65°C. In certain embodiments, the temperature for incubation of step (b) is 60°C, 61°C, 62°C, 63°C, 64°C, or 65°C.

The time for incubation of step (b) of the present disclosure refers to a time sufficient to observe significant amplification of the target nucleic acid by the LAMP reaction, which may be adjusted depending upon the amount of target nucleic acids expected to be included in the sample, etc. As an example, the time for incubation of step (b) may be increased to improve the sensitivity of the method.

According to one embodiment of the present disclosure, the time for incubation of step (b) is any time selected from 10 to 30 minutes, any time selected from 10 to 25 minutes, any time selected from 10 to 20 minutes, any time selected from 10 to 15 minutes, any time selected from 15 to 30 minutes, any time selected from 15 to 25 minutes, any time selected from 15 to 20 minutes, any time selected from 20 to 30 minutes, any time selected from 20 to 25 minutes, or any time selected from 25 to 30 minutes. In certain embodiments, the time for incubation of step (b) is 20 minutes or 30 minutes.

During or after step (b), the method of the present disclosure may further comprise detecting amplified products (target nucleic acids).

According to an embodiment, the detection of the amplified products may be performed by various detection devices known in the art. The detection device used may vary depending on the type of change occurring from the amplified products. For example, when a change in a fluorescence signal occurs from amplified products as a target nucleic acid is amplified, a detector for detecting the fluorescence signal may be used.

According to an embodiment, the detection of the amplified products may be performed in an end-point manner or in a real-time manner.

According to an embodiment, the detection of the amplified products may be performed at a predetermined time interval during step (b), for example, every 30 sec, 1 min, 1 min 30 sec, 2 min, 3 min, 4 min, or 5 min during step (b).

According to an embodiment, the amplified products may be detected using a fluorescent dye, such as SYBR Green 1, Eva green *(e.g.,* LAMP Fluorescent Dye 50x, New England BioLabs Inc.), a probe oligonucleotide labeled with a fluorescent dye, such as molecular beacon, indicator change, precipitate generation, turbidity, agarose gel DNA laddering, phosphorescence measurement, radiation measurement, capillary electrophoresis, a DNA chip, and the like.

According to an embodiment, the amplified products may be detected by a color change of a reaction solution depending upon DNA synthesis. In this case, the LAMP reagent may include an appropriate indicator, which includes, for example, color-changing dyes in response to pH change, such as phenol red or bromothymol blue, or color-changing dyes in response to magnesium ion concentration, such as hydroxy naphthalene blue (HNB). In addition, the target nucleic acid may be quantified or qualified by comparison with the amplified products of the positive and/or negative control sample.

According to an embodiment, isothermal amplification may be monitored in real time using a nucleic acid detection device capable of detecting a fluorescent dye during the LAMP reaction, without additional electrophoresis or addition of SYBR Green I after the reaction, and the amplification of the target nucleic acid may also be confirmed through annealing peak.

According to an embodiment, the amplified products may be detected by a direct or indirect method. In the direct method, a detectably labeled primer (or probe) may be used, and the amplification of the target nucleic acid may be confirmed by a signal emitted from the labeled primer after specific binding to the target nucleic acid in real-time. In the indirect detection method, a labeled probe capable of binding to the amplified target nucleic acid may be used. As used herein, the term "detectably labeled" means labeling with a substance capable of emitting a detectable signal using any suitable method, such as spectroscopic, optical, photochemical, biochemical, enzymatic, electrical, and/or immunochemical methods. Examples of substances used for such detectably labeling may include fluorescent moieties, chemiluminescent moieties, bioluminescent moieties, magnetic particles, enzymes, substrates, radioactivity, and chromophore substances.

According to an embodiment, a label for detection may include a fluorescent label, a luminescent label, a chemiluminescent label, an electrochemical label, and a metal label. The label may be used in the form of an unlinked label, such as an intercalating dye. Alternatively, a single label, or an interactive dual label comprising a donor molecule and an acceptor molecule may be used in the form linked to one or more oligonucleotides.

The single label may include a fluorescent label, a luminescent label, a chemiluminescent label, or an electrochemical label, and a metal label. The single label provides different signals (e.g., different signal intensities) depending on whether the single label is present on a double strand or a single strand.

According to certain embodiments of the present disclosure, the single label may be a fluorescent label.

The single label may be linked to an oligonucleotide by various methods. For example, the label is linked to the primer (or probe) via a spacer comprising carbon atoms (e.g., 3-carbon spacer, 6-carbon spacer, or 12-carbon spacer).

The interactive label system includes a signal generation system in which energy is transferred non-radioactively between a donor molecule (reporter molecule) and an acceptor molecule (quencher molecule).

As a representative example of the interactive label system, a fluorescence resonance energy transfer (FRET) label system includes a fluorescent reporter molecule (donor molecule) and a quencher molecule (acceptor molecule). In FRET, the energy donor is fluorescent, but the energy acceptor may be fluorescent or non-fluorescent. In another form of an interactive label system, the energy donor is non-fluorescent, *e.g*., chromophore and the energy acceptor is fluorescent. In another form of an interactive label system, the energy donor is luminescent, *e.g*., bioluminescent, chemiluminescent, or electrochemiluminescent and the acceptor is fluorescent.

The interactive dual label includes a pair of labels providing a detectable signal based on contact-mediated quenching (Salvatore et al., Nucleic Acids Research, 2002 (30) no. 21 e122 and Johansson et al., J. AM. CHEM. SOC 2002 (124) pp 6950-6956). The interactive label system includes all or any case that includes a signal change due to an interaction between at least two molecules **(*e.g*.,** dyes).

Reporter molecules and quencher molecules useful as single or dual labels may include any molecule known in the art. Examples thereof may include: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™} (531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™} (576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red (615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). The number of parentheses is a maximum emission wavelength expressed in nanometers.

Appropriate reporter-quencher pairs are disclosed in Pesce et el., editors, Fluorescence Spectroscopy (Marcel Dekker, New York, 1971); White et el., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996); U.S Pat. Nos. 3,996,345 and 4,351,760.

Each of the reporter molecule and the quencher molecule may be fluorescent. Alternatively, the reporter molecule may be fluorescent, and the quencher molecule may be non-fluorescent. For example, a non-fluorescent dark quencher capable of quenching fluorescence of a wide range wavelength or a specific wavelength can be used in the present disclosure. When the quencher molecule is fluorescent, the target nucleic acid may be detected from a signal change of the fluorescent quencher molecule.

The reporter molecule and the quencher molecule may be linked to an oligonucleotide according to conventional methods. For example, the reporter molecule and the quencher molecule can be linked to the probe via a spacer comprising at least three carbon atoms (*e.g*., a 3-carbon spacer, a 6-carbon spacer, a 9-carbon spacer, or a 12-carbon spacer).

According to an embodiment of the present disclosure, conventionally known fluorescence detection methods may be used to detect the target nucleic acid amplified by the LAMP reaction. For example, a real-time measurement method using two-stranded assimilating probe specific to a target nucleic acid based on the principle of fluorescence resonance energy transfer (FRET) may be used (WO2011/163425). According to the method, a reporter molecule is attached to the 5' end of one strand of the assimilating probe, and a quencher molecule is attached to the 3' end of the other strand of the assimilating probe. As another example, similar to the assimilating probe, a method of using a fluorescently labeled primer/probe and a quencher labeled probe may be used (WO2013/065574).

According to an embodiment of the present disclosure, the detection of the amplified products may be performed by measuring fluorescence.

According to an embodiment of the present disclosure, the LAMP reagent may comprise an oligonucleotide comprising a fluorescent molecule and an oligonucleotide comprising a quencher molecule.

According to an embodiment of the present disclosure, one of the six LAMP primers included in the LAMP reagent may be used as an oligonucleotide comprising a fluorescent molecule or an oligonucleotide comprising a quencher molecule, and for example, FIP, BIP, LF, or LB labeled with a fluorescent molecule or a quencher molecule may be designed as an oligonucleotide comprising a fluorescent molecule or an oligonucleotide comprising a quencher molecule.

According to an embodiment of the present disclosure, the LAMP reagent may be a reagent for amplifying a plurality of target nucleic acids, wherein the plurality of target nucleic acids may be 2 to 5, specifically 2 to 4, and more specifically 3.

In order to amplify the target nucleic acid, the method of the present disclosure provides a LAMP reagent for each target nucleic acid. For example, when there are three target nucleic acids, a first reagent for amplifying a first target nucleic acid, a second reagent for amplifying a second target nucleic acid, and a third reagent for amplifying a third target nucleic acid are provided. The plurality of LAMP reagents with respect to the target nucleic acids may differ only in a set of primers and/or a probe, and the remaining components may be the same.

The LAMP reagent may be contained in one reaction vessel to simultaneously amplify the plurality of targets. Such simultaneous amplification of a plurality of target nucleic acids may be referred to as a "multiplex LAMP method".

The effect of the method according to the present disclosure is described in terms of improving, enhancing, or promoting "amplification of a target nucleic acid" or "specific amplification" and suppressing, lowering, or reducing "amplification of a non-target nucleic acid" or "non-specific amplification".

As used herein, "amplification of a target nucleic acid" or "specific amplification" refers to amplification of the target nucleic acid by proper interaction between the set of primers used in the method of the present disclosure and the target nucleic acid to be detected.

In contrast, "amplification of a non-target nucleic acid" or "non-specific amplification" as used herein refers to amplification by an interaction between the set of primers used in the method of the present disclosure and a non-target nucleic acid or by an interaction between primers.

Such non-specific amplification may be represented by amplification in no template control (NTC).

As used herein, the term "NTC (No template control)" refers to performing an amplification reaction using a LAMP reagent in the absence of a target nucleic acid. Thus, the amplification in NTC may be considered to be non-specific amplification rather than specific amplification of the target nucleic acid.

According to the present disclosure, adding a beta-hydroxy acid, such as L-serine or L-carnitine, to a LAMP reaction in the presence of a target nucleic acid increases the rate of amplification of the target nucleic acid, and adding L-serine or L-carnitine to a LAMP reaction in the absence of the target nucleic acid (NTC) decreases the non-specific amplification signal. In particular, appropriate concentrations of L-serine or L-carnitine showed excellent effects, but other concentrations did not.

Non-specific amplification signals in the LAMP reaction are likely to occur in the presence of excess primers, polymerase, and/or magnesium. Thus, it is expected that the method of the present disclosure is particularly useful in reactions where excess components for LAMP are used.

### II. LAMP reagent comprising beta-hydroxy acid

In another aspect, the present disclosure provides reagent for loop mediated isothermal amplification (LAMP), the reagent comprising:
(i) a set of primers comprising an F3 primer, a B3 primer, a forward inner primer (FIP), and a backward inner primer (BIP);
(ii) a nucleic acid polymerase;
(iii) dNTPs;
(iv) beta-hydroxy acid; and
(v) a buffer.

Since Aspect II overlaps with Aspect I of the present disclosure, common descriptions between them is omitted in order to avoid undue redundancy.

### III. Method for reducing non-specific amplification signals

In another aspect, the present disclosure provides a method for reducing a non-specific amplification signal in amplification of a target nucleic acid, the method comprising:
(a) providing a reagent for loop-mediated isothermal amplification (LAMP), the reagent comprising:
   (i) a set of primers comprising an F3 primer, a B3 primer, a forward inner primer (FIP), and a backward inner primer (BIP);
   (ii) a nucleic acid polymerase;
   (iii) dNTPs;
   (iv) beta-hydroxy acid; and
   (v) a buffer; and
(b) incubating the LAMP reagent with a target nucleic acid at an isothermal temperature to amplify the target nucleic acid, wherein the LAMP reagent comprising beta-hydroxy acid reduces non-specific amplification as compared to a LAMP reagent not containing beta-hydroxy acid.

Since Aspect III overlaps with Aspect I of the present disclosure, common descriptions between them is omitted in order to avoid undue redundancy.

The present invention will now be described in further detail by examples. It would be obvious to those skilled in the art that these examples are intended to be more concretely illustrative and the scope of the present invention as set forth in the appended claims is not limited to or by the examples.

### [Mode for Invention]

### Example 1: Effect of beta-hydroxy acid on amplification of target nucleic acid in LAMP reaction

To examine the effect of the addition of beta-hydroxy acid on the LAMP reaction, LAMP reagents comprising L-serine or L-carnitine as beta-hydroxy acid were prepared. For comparison, LAMP reagents comprising an additive known in the art, *i.e.,* betaine, DMSO, or a mixture of formamide (FA) and N,N-dimethylformamide (DMF), respectively, were prepared, and a LAMP reagent not containing any beta-hydroxy acid or additive described above was also prepared.

### <1-1> Preparation of a LAMP reagent not containing any beta-hydroxy acid or additive

2.5 ul of a primer mix (10x primer mix) consisting of 0.2 uM of F3 and B3 primers (SEQ ID NOs: 1 and 2), 1.6 uM of FIP and BIP primers (SEQ ID NOs: 3 and 4), and 0.4 uM of LF and LB primers (SEQ ID NOs: 5 and 6) which were designed to hybridize to M13mp18a single-stranded DNA as a template was mixed with 0.5 ul of a LAMP fluorescent dye (NEB), 1 ul (8 Unit/rxn) of *Bst* DNA polymerase, Large fragment (NEB), 2.5 ul of 10x buffer, 1.5 ul of 100 mM MgSO₄, and 3.5 ul of 10 mM dNTP mix to prepare a LAMP reagent of Example <1-1> in a final volume of 25 ul.

### <1-2> Preparation of a LAMP reagent containing citrate

20 mM of citrate was added to the reagent of Example <1-1> to prepare a LAMP reagent of Example <1-2>.

### <1-3> Preparation of a LAMP reagent containing L-serine

20 mM of L-serine was added to the reagent of Example <1-1> to prepare a LAMP reagent of Example <1-3>.

### <1-4> Preparation of a LAMP reagent containing L-carnitine

2 mM of L-carnitine was added to the reagent of Example <1-1> to prepare a LAMP reagent of Example <1-4>.

### <1-5> Preparation of a LAMP reagent containing betaine

1 M of betaine was added to the reagent of Example <1-1> to prepare a LAMP reagent of Example <1-5>.

### <1-6> Preparation of a LAMP reagent containing DMSO

7.5% of DMSO was added to the reagent of Example <1-1> to prepare a LAMP reagent of Example <1-6>.

### <1-7> Preparation of a LAMP reagent containing FA and DMF in combination

1.8 M of FA and DMF (FA 1.385 M and DMF 0.415 M) in combination were added to the reagent of Example <1-1> to prepare a LAMP reagent of Example <1-7>.

Each of the LAMP reagents prepared in Examples <1-1> to <1-7> was mixed with 10⁸ cp/rxn of M13mp18 single-stranded DNA (NEB) as a target nucleic acid to prepare a mixture.

The mixture was incubated at 65°C for 90 minutes in a CFX96 instrument. Fluorescence generated from the target nucleic acid amplified during the incubation was measured at 1-minute intervals to obtain an amplification curve. Then, a threshold of 2500 was applied to the amplification curve to calculate a Ct value (Time to positive; min).

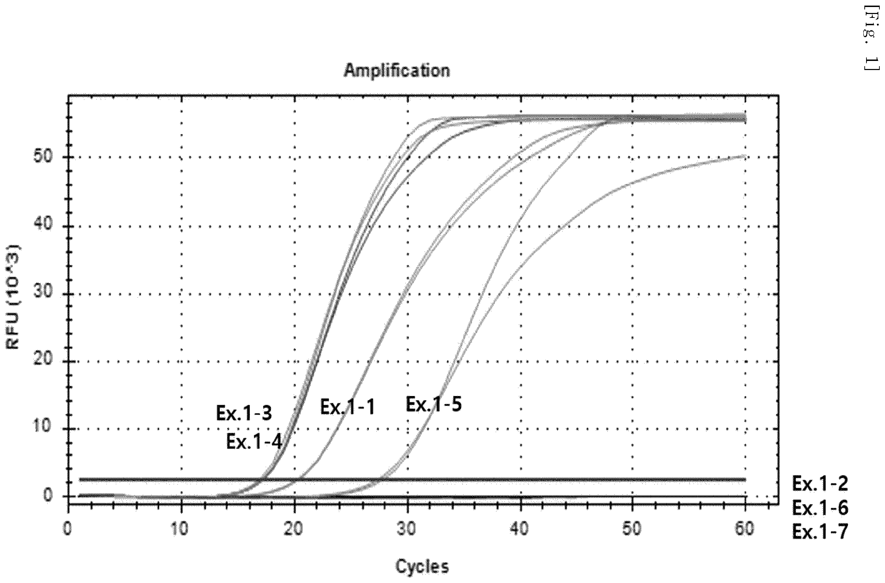

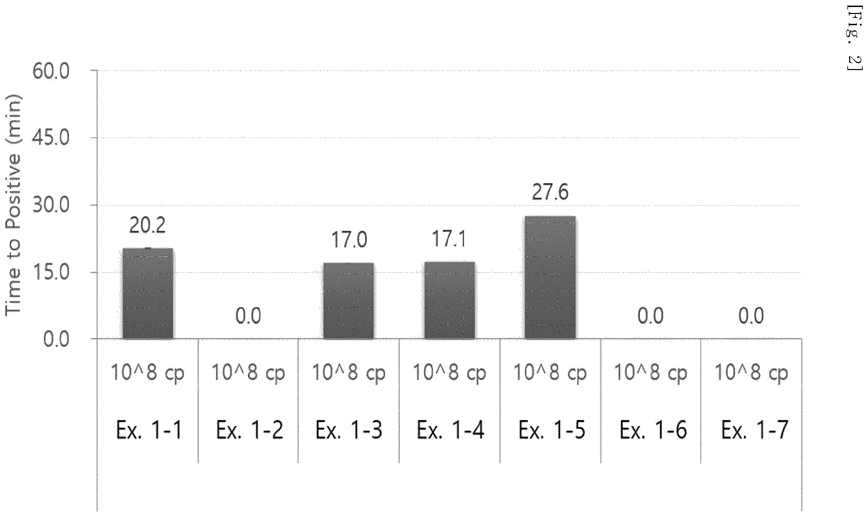

The resulting amplification curves and Ct values calculated therefrom are shown in FIGS. 1 and 2, respectively.

As shown in FIGS. 1 and 2, in the presence of the target nucleic acid, the LAMP reaction using the reagent of Example <1-1> not containing beta-hydroxy acids or additives exhibited a Ct value of 20.2, whereas the LAMP reaction using the reagent of Example <1-3> containing L-serine exhibited a Ct value of 17.0, and the LAMP reaction using the reagent of Example <1-4> containing L-carnitine exhibited a Ct value of 17.1. This demonstrates that the addition of beta-hydroxy acid such as L-serine or L-carnitine enhances the rate of the LAMP reaction.

On the other hand, in the presence of the target nucleic acid, the LAMP reaction using the reagent of Example <1-5> containing betaine conventionally known in the art exhibited a Ct value of 27.6, and the LAMP reaction using the reagents of Example <1-2> containing citrate, Example <1-6> containing DMSO, and Example <1-7> containing FA/DMF did not exhibit amplification of the target nucleic acid (Ct value not determined). This shows that betaine, citrate, DMSO, and FA/DMF inhibited the LAMP reaction.

The above results confirmed that the addition of beta-hydroxy acid such as L-serine or L-carnitine to the LAMP reaction can improve the amplification of the target nucleic acid.

### Example 2: Effect of beta-hydroxy acid against non-specific amplification in the LAMP reaction

To examine the effect of the addition of beta-hydroxy acid against non-specific amplification in the LAMP reaction, LAMP reagents comprising L-serine or L-carnitine as beta-hydroxy acid were prepared, respectively. For comparison, a LAMP reagent containing citrate and a LAMP reagent not containing any beta-hydroxy acid or citrate were also prepared.

### <2-1> Preparation of a LAMP reagent not containing any beta-hydroxy acid or citrate

2.5 ul of a primer mix (10x primer mix) consisting of 0.2 uM of F3 and B3 primers (SEQ ID NOs: 1 and 2), 1.6 uM of FIP and BIP primers (SEQ ID NOs: 3 and 4), and 0.4 uM of LF and LB primers (SEQ ID NOs: 5 and 6) which were designed to hybridize to template M13mp18 single-stranded DNA as a template was mixed with 12.5 ul of a commercially available master mix (Elpis-Biotech, Cat no. EBT-1760) was mixed to prepare a LAMP reagent of Example <2-1> in a final volume of 25 ul.

### <2-2> Preparation of a LAMP reagent containing 0.2 M citrate

0.2 M citrate was added to the reagent of Example <2-1> to prepare a LAMP reagent of Example <2-2>.

### <2-3> Preparation of a LAMP reagent containing 0.02 M citrate

0.02 M citrate was added to the reagent of Example <2-1> to prepare a LAMP reagent of Example <2-3>.

### <2-4> Preparation of a LAMP reagent containing 0.2 M L-serine

0.2 M of L-serine was added to the reagent of Example <2-1> to prepare a LAMP reagent of Example <2-4>.

### <2-5> Preparation of a LAMP reagent containing 0.02 M L-serine

0.02 M of L-serine was added to the reagent of Example <2-1> to prepare a LAMP reagent of Example <2-5>.

### <2-6> Preparation of a LAMP reagent containing 0.2 M L-carnitine

0.2 M of L-carnitine was added to the reagent of Example <2-1> to prepare a LAMP reagent of Example <2-6>.

### <2-7> Preparation of a LAMP reagent containing 0.02 M L-carnitine

0.02 M of L-carnitine was added to the reagent of Example <2-1> to prepare a LAMP reagent of Example <2-7>.

### <2-8> Preparation of a LAMP reagent containing 0.002 M L-carnitine

0.002 M of L-carnitine was added to the reagent of Example <2-1> to prepare a LAMP reagent of Example <2-8>.

In order to examine whether the LAMP reagents of Examples <2-1> to <2-8> inhibit non-specific amplification in no template control (NTC), the LAMP reagents (without target nucleic acid) were incubated at 65°C for 90 minutes in a CFX96 instrument. Fluorescence was measured at 1-minute intervals during the incubation to obtain a non-specific amplification curve. Then, a threshold of 2500 was applied to the non-specific amplification curve to calculate a time to positive (Ct) value.

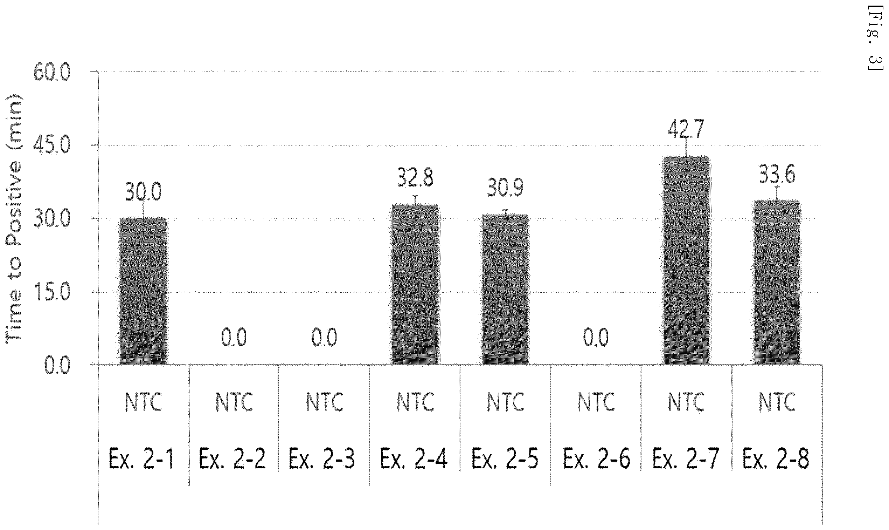

The Ct values are shown in FIG. 3.

As shown in FIG. 3, in the absence of the target nucleic acid, the LAMP reaction using the LAMP reagent of Example <2-1> not containing any beta-hydroxy acid or citrate showed non-specific amplification with a Ct value of 30 cycles. On the other hand, in the absence of the target nucleic acid, the LAMP reactions using the LAMP reagents of Examples <2-2> and <2-3> containing 0.2 M or 0.02 M citrate did not show non-specific amplification, nor specific amplification of the target nucleic acid as demonstrated in Example 1. On the other hand, in the absence of the target nucleic acid, the LAMP reactions using the LAMP reagents of Examples <2-4> and <2-5> containing 0.2 M or 0.02 M of L-serine or Examples <2-5>, <2-6>, and <2-7> containing 0.2 M, 0.02 M, or 0.002 M of L-carnitine showed Ct values of 32.8, 30.9, 0, 42.7, and 33.6, which indicated no or inhibited non-specific amplification compared to the reagent of Example <2-1>.

The results show that the addition of beta-hydroxy acid such as L-serine or L-carnitine to the LAMP reaction can inhibit non-specific amplification.

### Example 3: Effect of concentration of beta-hydroxy acid

In order to examine the optimum concentration of beta-hydroxy acid in the LAMP reaction, LAMP reagents containing various concentrations of L-serine or L-carnitine were prepared.

2.5 ul of a primer mix (10x primer mix) consisting of 0.2 uM of F3 and B3 primers (SEQ ID NOs: 1 and 2), 1.6 uM of FIP and BIP primers (SEQ ID NOs: 3 and 4), and 0.4 uM of LF and LB primers (SEQ ID NOs: 5 and 6) which were designed to hybridize to M13mp18a single-stranded DNA as a template was mixed with 12.5 ul of a commercially available master mix (Elpis-Biotech, Cat no. EBT-1760), and 0, 10, 20, 50, 100, or 200 mM of serine or 0, 0.5, 1, 10, or 20 mM of carnitine as beta-hydroxy acid to prepare LAMP reagents in a final volume of 25 ul.

The LAMP reagents as prepared above was each mixed with 10³ cp/rxn of M13mp18 single-stranded DNA (NEB) as a target nucleic acid to prepare a mixture. In addition, in order to examine non-specific amplification, the LAMP reagent not containing the target nucleic acid was used as no template control (NTC).

The mixture or NTC was incubated at 65°C for 90 minutes in a CFX96 instrument. During the incubation, fluorescence was measured at 1-minute intervals to obtain an amplification curve. Then, a threshold value of 2500 was applied to the amplification curve to calculate a Ct value.

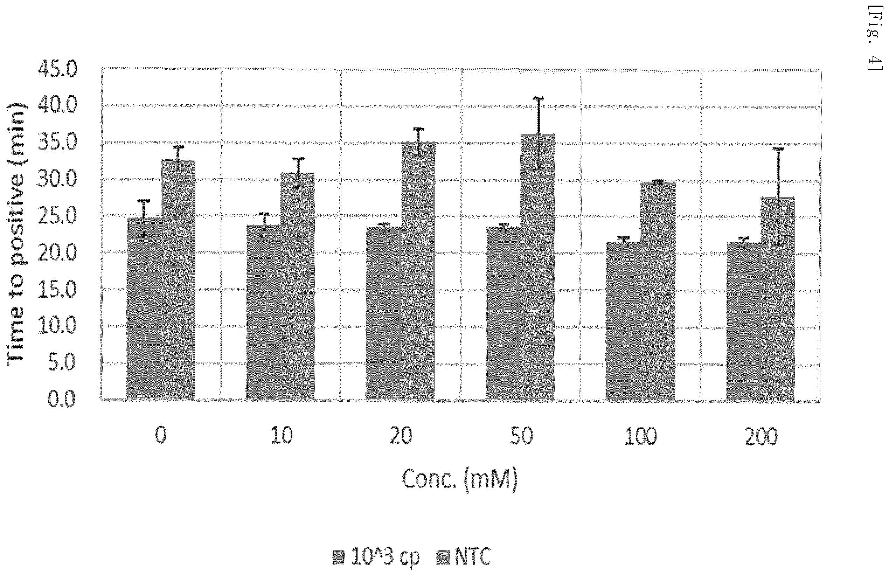

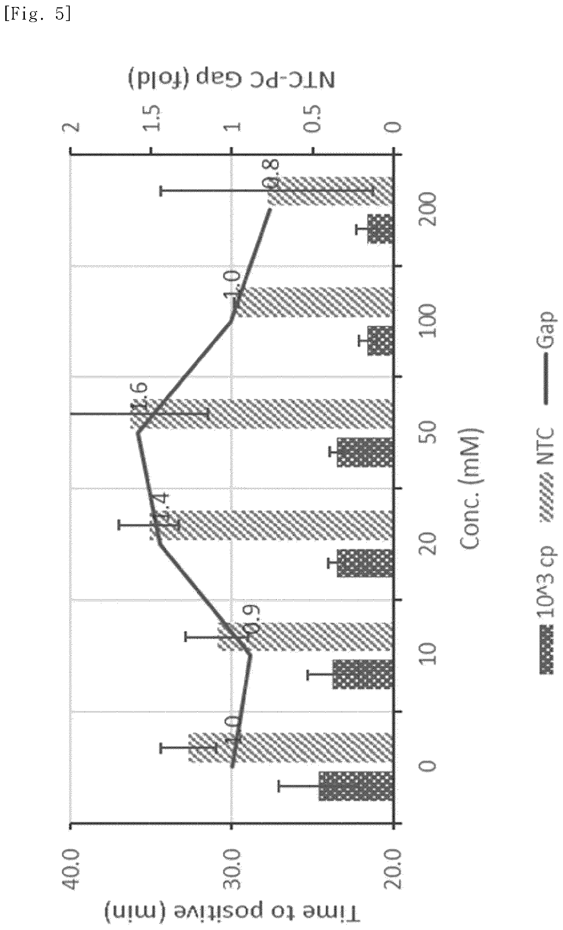

The amplification results of the target nucleic acid or NTC using the LAMP reagents containing L-serine are shown in FIG. 4. Further, the fold increase of the difference in Ct value between amplification of NTC and amplification of the target nucleic acid for reactions using 10, 20, 50, 100, or 200 mM L-serine, relative to the difference in Ct value between amplification of NTC and amplification of the target nucleic acid for reactions using 0 mM L-serine is shown in FIG. 5.

As shown in FIGS. 4 and 5, the addition of 20 mM and 50 mM L-serine could increase the difference between amplification of the target nucleic acid and amplification of NTC by 1.4 times and 1.6 times, respectively, compared to no addition of L-serine. In contrast, the addition of 10 mM L-serine had no significant difference as compared to no addition of L-serine, and the addition of 100 mM and 200 mM L-serine resulted in decreased amplification of the target nucleic acid and increased amplification of NTC compared to no addition of serine.

Accordingly, the above results demonstrate that it is preferred to use L-serine at an appropriate concentration, *i.e.,* a concentration between 20 mM and 50 mM.

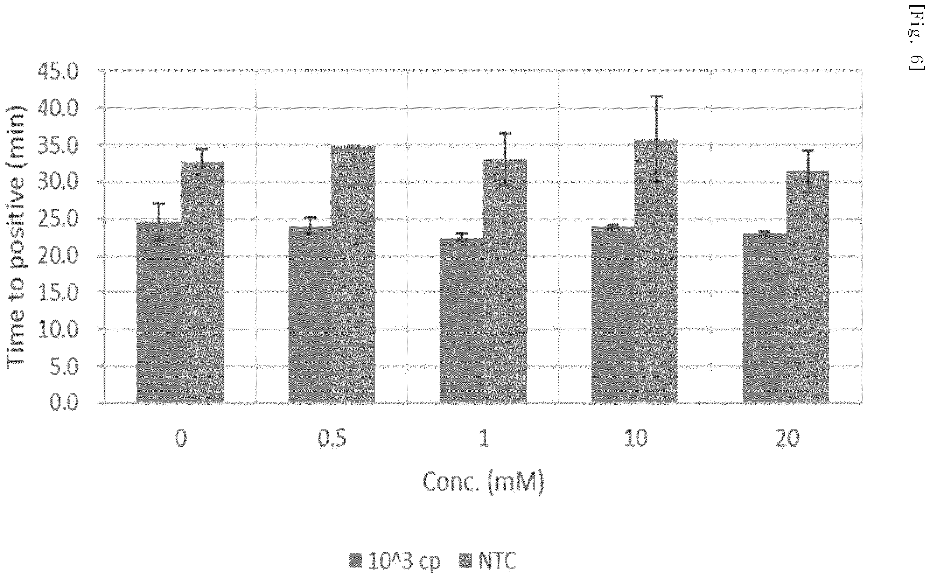

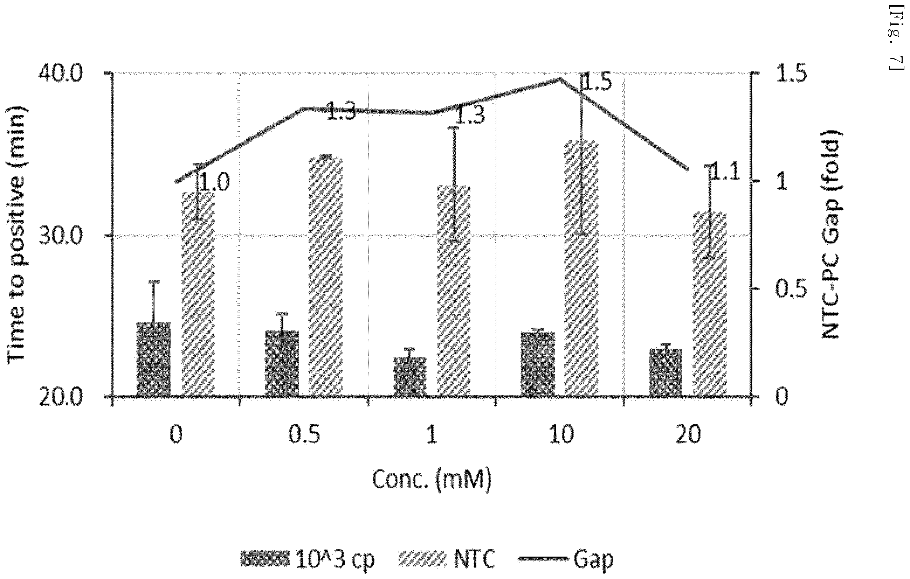

Meanwhile, the amplification results of the target nucleic acid or NTC using the LAMP reagents containing L-carnitine are shown in FIG. 6. Further, the fold increase of the difference in Ct value between amplification of NTC and amplification of the target nucleic acid for reactions using 0.5, 1, 10, or 20 mM L-carnitine, relative to the difference in Ct value between amplification of NTC and amplification of the target nucleic acid for reactions using 0 mM L-carnitine is shown in FIG. 7.

As shown in FIGS. 6 and 7, the addition of 0.5 mM, 1 mM, and 10 mM L-carnitine could increase the difference between amplification of the target nucleic acid and amplification of NTC by 1.3 times, 1.3 times and 1.5 times, respectively, compared to the no addition of L-carnitine. In contrast, the addition of 20 mM L-carnitine resulted in decreased amplification of the target nucleic acid and increased amplification of NTC as compared to no addition of L-carnitine.

Accordingly, the results demonstrate that it is preferred to use L-carnitine at an appropriate concentration, *i.e.,* a concentration of 0.5 mM to 10 mM.

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A method for amplifying a target nucleic acid, the method comprising:
(a) providing a loop-mediated isothermal amplification (LAMP) reagent, the reagent comprising:
(i) a set of primers comprising an F3 primer, a B3 primer, a forward inner primer (FIP), and a backward inner primer (BIP);
(ii) a nucleic acid polymerase;
(iii) dNTPs;
(iv) beta-hydroxy acid; and
(v) a buffer; and
(b) incubating the LAMP reagent with a target nucleic acid at an isothermal temperature to amplify the target nucleic acid.

2. The method of claim 1, wherein the beta-hydroxy acid is a bidentate chelator compound.

3. The method of claim 2, wherein the beta-hydroxy acid is selected from the group consisting of serine, carnitine, threonine, and combinations thereof.

4. The method of claim 3, wherein each of the serine, carnitine and threonine is in an L-isomeric form.

5. The method of claim 4, wherein the beta-hydroxy acid is L-serine, and the L-serine is comprised at a concentration of 15 to 80 mM.

6. The method of claim 4, wherein the beta-hydroxy acid is L-carnitine, and the L-carnitine is comprised at a concentration of 0.2 to 20 mM.

7. The method of claim 4, wherein the beta-hydroxy acid is L-threonine, and the L-threonine is comprised at a concentration of 15 to 80 mM.

8. The method of claim 3, wherein the beta-hydroxy acid is a combination of L-serine and L-carnitine.

9. The method of claim 1, wherein the nucleic acid polymerase has strand displacement activity and lacks 5' to 3' exonuclease activity.

10. The method of claim 1, wherein the nucleic acid polymerase is selected from the group consisting of a full-length or large fragment of *Bst* (*Bacillus stearothermophilus*) DNA polymerase, a full-length or large fragment of *Bsm* (*Bacillus smithii*) DNA polymerase, a full-length or large fragment of *Bsu* (*Bacillus subtilis*) DNA polymerase, a full-length or large fragment of *Sau* (*Staphylococcus aureus*) DNA polymerase, and a mutant thereof.

11. The method of claim 1, wherein the set of primers further comprises one or both of a loop forward (LF) primer and a loop backward (LB) primer.

12. The method of claim 1, which increases amplification efficiency of the target nucleic acid while reducing non-specific amplification as compared to using a LAMP reagent that does not include beta-hydroxy acid.

13. The method of claim 1, wherein the target nucleic acid is 3 to 5.

14. The method of claim 1, wherein the target nucleic acid is RNA.

15. The method of claim 14, which further comprises reverse transcribing the target RNA into target DNA.

16. A reagent for loop mediated isothermal amplification (LAMP), the reagent comprising:
(i) a set of primers comprising an F3 primer, a B3 primer, a forward inner primer (FIP), and a backward inner primer (BIP);
(ii) a nucleic acid polymerase;
(iii) dNTPs;
(iv) beta-hydroxy acid; and
(v) a buffer.

17. The reagent of claim 16, wherein the beta-hydroxy acid is a bidentate chelator compound.

18. The reagent of claim 17, wherein the beta-hydroxy acid is selected from the group consisting of serine, carnitine, threonine, and combinations thereof.

19. The reagent of claim 18, wherein the serine, carnitine and threonine are in an L-isomeric form.

20. The reagent of claim 19, wherein the beta-hydroxy acid is L-serine, and the L-serine is comprised at a concentration of 15 to 80 mM.

21. The reagent of claim 19, wherein the beta-hydroxy acid is L-carnitine, and wherein the L-carnitine is comprised at a concentration of 0.2 to 20 mM.

22. The reagent of claim 19, wherein the beta-hydroxy acid is L-threonine, and the L-threonine is comprised at a concentration of 10 to 50 mM.

23. The reagent of claim 18, wherein the beta-hydroxy acid is a combination of L-serine and L-carnitine.

24. The reagent of claim 16, wherein the nucleic acid polymerase has strand displacement activity and lacks 5' to 3' exonuclease activity.

25. The reagent of claim 16, wherein the nucleic acid polymerase is selected from the group consisting of a full-length or large fragment of *Bst* (*Bacillus stearothermophilus*) DNA polymerase, a full-length or large fragment of *Bsm* (*Bacillus smithii*) DNA polymerase, a full-length or large fragment of *Bsu* (*Bacillus subtilis*) DNA polymerase, a full-length or large fragment of *Sau* (*Staphylococcus aureus*) DNA polymerase, and a mutant thereof.

26. The reagent of claim 16, wherein the primer set further comprises one or both of a loop forward (LF) primer and a loop backward (LB) primer.

27. The reagent of claim 16, wherein the reagent increases the amplification rate of the target nucleic acid while reducing non-specific amplification as compared to a LAMP reagent not containing beta-hydroxy acid.

28. The reagent of claim 16, wherein the target nucleic acid is 3 to 5.

29. The reagent of claim 16, wherein the target nucleic acid is RNA.

30. A method for reducing a non-specific amplification signal in amplification of a target nucleic acid, the method comprising:
(a) providing a reagent for loop-mediated isothermal amplification (LAMP), the reagent comprising:
(i) a set of primers comprising an F3 primer, a B3 primer, a forward inner primer (FIP), and a backward inner primer (BIP);
(ii) a nucleic acid polymerase;
(iii) dNTPs;
(iv) beta-hydroxy acid; and
(v) a buffer; and
(b) incubating the LAMP reagent with a target nucleic acid at an isothermal temperature to amplify the target nucleic acid, wherein the LAMP reagent comprising beta-hydroxy acid reduces non-specific amplification as compared to a LAMP reagent not containing beta-hydroxy acid.
